# EUROPEAN PATENT APPLICATION

(11) **EP 3 284 400 A2**
(43) Date of publication of application: **21.02.2018**
(21) Application number: 17162105.5
(22) Date of filing: 02.10.2015
(51) Int. Cl.: A61B 5/00, A61B 5/08, A61B 5/117, A61M 15/00

(54) **BREATHPRINT SENSOR SYSTEMS, SMART INHALERS AND METHODS FOR PERSONAL IDENTIFICATION**

(30) Priority: 17.10.2014 US 201462065465 P; 02.07.2015 US 201514791049
(62) Divisional of application: 15784196.6
(71) Applicant: QUALCOMM Incorporated, San Diego, CA 92121-1714 (US)
(72) Inventor: SEZAN, Muhammed Ibrahim, San Diego, CA 92121-1714 (US); DANTSKER, Eugene, San Diego, CA 92121-1714 (US); LONDERGAN, Ana Rangelova, San Diego, CA 92121-1714 (US); HOFFMAN, Paul Robert, San Diego, CA 92121-1714 (US)
(74) Representative: Dunlop, Hugh Christopher

(57) **Abstract**

Smart inhaler systems for compliance-based delivery of one or more drugs to a person by inhalation. The smart inhaler systems include a breathprint sensor system configured to process gases produced by the person, an inhaler apparatus configured to deliver the one or more drugs to the person by inhalation, and a control system. The control system is configured to receive drug dosage data and time data and control an operation of the inhaler apparatus according to at least the drug dosage data and the time data. Methods for operating smart inhaler systems and computer-readable media for implementing the methods are also disclosed.

## Description

### PRIORITY DATA

This patent document claims priority to and benefit of U.S. Patent Application No. 14/791,049, entitled: BREATHPRINT SENSOR SYSTEMS, SMART INHALERS AND METHODS FOR PERSONAL IDENTIFICATION, by Sezan et al., filed July 2, 2015, which claims priority to and benefit of U.S. Provisional Patent Application No. 62/065,465, entitled: BREATHPRINT SENSOR SYSTEMS, SMART INHALERS AND METHODS FOR PERSONAL IDENTIFICATION, by Sezan et al., filed October 17, 2014. Both U.S. Patent Application No. 14/791,049 and U.S. Provisional Patent Application No. 62/065,465 are hereby incorporated by reference in their entireties and for all purposes.

### TECHNICAL FIELD

This disclosure relates generally to personal identification. More specifically, it relates to verifying a person's identity using gases produced by the person, such as gases in the person's breath.

### DESCRIPTION OF THE RELATED TECHNOLOGY

Various conventional ways exist for verifying a person's identity: requesting a person to provide a password or other information of which only the person has knowledge; checking a photo ID card bearing the person's picture and associated information such as name, gender and age; taking a biometric sample (e.g., a finger print or a retina scan) from the person that is specific to the person; examining a unique token given only to the person, etc. These conventional techniques are still widely used and can be cumbersome and time-consuming to implement.

Conventional healthcare practices have experienced various problems in drug administration: the patient might receive the wrong drug, the right drug might be delivered to the wrong patient, or the right drug might be administered in the wrong dose. One way to view the problems in drug administration is compliance: whether or not the patient is taking the drug as prescribed. Conventional healthcare organizations employ personnel to implement manual checks and balances, for instance, using clipboards to enter drug information into physical charts for tracking and analysis. These conventional techniques are inefficient and prone to human errors. The effectiveness of these conventional techniques is further diminished when the patient is in a home care setting rather than a clinical setting since the infrastructure for ensuring compliance is not available at home. It is thus desirable to have a reliable system to provide and use compliance information.

### SUMMARY

Some implementations of systems, methods, apparatuses, and computer-readable media of the disclosure have several aspects, no single one of which is solely responsible for the desirable attributes disclosed herein.

Some aspects of the present disclosure provide a breathprint sensor for verifying the identity of a particular person such as a patient. Some aspects of the disclosure relate to a smart inhaler system using the breathprint sensor to assist in delivery of drugs to the patient through inhalation.

One aspect of the subject matter described in this disclosure can be implemented in a breathprint sensor system for verifying the identity of a person using gases produced by the person, e.g., through exhalation, secretion, discharge, emission, and emanation. The breathprint sensor system includes one or more first sensors having first response characteristics to compounds in gases. The one or more first sensors are configured to output sensor data representing the first response characteristics. The breathprint sensor system also includes one or more processors in communication with the one or more first sensors. The one or more processors are configured to: receive a set of test data provided by the one or more first sensors based on an exposure of the one or more first sensors to gases during a test phase; and determine whether the set of test data verifies the identity of the person.

In some implementations, the breathprint sensor system further includes one or more second sensors for identifying biological or environmental conditions. The one or more second sensors have second response characteristics to compounds in gases. The one or more second sensors are configured to output supplemental sensor data representing the second response characteristics. The one or more processors of breathprint sensor system are in further communication with the one or more second sensors. The one or more processors are further configured to: (a) receive a set of supplemental sensor data provided by the one or more second sensors based on an exposure of the one or more second sensors to the gases produced by the person during the test phase; (b) identify a biological or environmental condition associated with the set of supplemental sensor data; and (c) provide information indicating the biological or environmental condition.

In some implementations, the breathprint sensor system determines whether a set of test data verifies the identity of the person by pre-processing the test data and using a pattern classifier to classify the pre-processed test data into one of a plurality of classes including: (i) identity verified when a pattern of the test data is recognized as belonging to the person, and (ii) identity not verified when a pattern of the test data is recognized as not belonging to the person. In some implementations, the pattern classifier includes a neural network pattern classifier.

In some implementations, the one or more processors are further configured to train the pattern classifier from one or more sets of training data. In some implementations, training the pattern classifier from one or more sets of training data involves: receiving one or more sets of positive training data provided by the one or more first sensors based on one or more exposures of the one or more first sensors to gases produced by the person during a training phase; providing the one or more sets of positive training data to the pattern classifier; and informing the pattern classifier that the one or more sets of positive training data belong to the person. In some implementations, training the pattern classifier from one or more sets of training data further involves: receiving one or more sets of negative training data provided by the one or more first sensors based on one or more exposures of the one or more first sensors to gases not produced by the person during a training phase; providing the one or more sets of negative training data to the pattern classifier; and informing the pattern classifier that the one or more sets of negative training data do not belong to the person.

In some implementations, one or more first sensors of the breathprint sensor systems described herein are made from sensor materials such as: conducting polymer, conducting polymer composites, intrinsically conducting polymers, and any combinations thereof. In some implementations, the one or more sensors are further configured to obtain the sensor data when a surface temperature of the one or more first sensors is: less than or equal to about 200 °C, less than or equal to about 100 °C, between about -50 °C and about 100 °C, or between about 0 °C and about 50 °C. In some implementations, the one or more first sensors of the breathprint sensor systems describe herein are further configured to obtain the sensor data when a surface temperature of the one or more first sensors is unmodulated or substantially constant. In some implementations, each sensor includes a polymer layer having a variable conductance based on exposure to volatile organic compounds (VOCs) in gases.

In some implementations, any of the breathprint sensor systems described herein further includes a memory configured to store the sensor data and/or information derived from the sensor data.

In some implementations, the one or more processors of any of the breathprint sensor systems are further configured to: derive a test feature vector from the test data through feature extraction. In some implementations, the one or more processors are further configured to: compare the test feature vector to a training feature vector derived from training data; and based on the comparison, determine whether the test data verifies the identity of the person.

In some implementations, the breathprint sensor system further includes: an inhaler apparatus adapted to deliver drugs to a person through inhalation when the inhaler apparatus is received by the person; a control system in communication with the breathprint sensor system and with the inhaler apparatus, and an interface system for inputting data to and/or outputting data from the breathprint sensor system, the inhaler apparatus, and/or the control system. The control system is configured to: receive information from the breathprint sensor system, the information indicating whether the person's identity is verified, and control an operation of the inhaler apparatus according to the received information.

Another aspect of the subject matter described in this disclosure can be implemented in a smart inhaler system for delivering drugs to a person by inhalation. The smart inhaler system includes a breathprint sensor system configured to verify an identity of the person using gases produced by the person; an inhaler apparatus adapted to deliver the drugs to the person through inhalation when the inhaler apparatus is received by the person; and a control system in communication with the breathprint sensor system and with the inhaler apparatus. The control system is configured to: receive information from the breathprint sensor system, the information indicating whether the person's identity is verified, and control an operation of the inhaler apparatus according to the received information. In some implementation of the smart inhaler system, controlling the operation of the inhaler apparatus according to the received information involves controlling a delivery of a drug according to the received information. In some implementations, the inhaler smart system further includes an interface system for inputting data to and/or outputting data from the breathprint sensor system, the inhaler apparatus, and/or the control system. In some implementations, the interface system includes a wireless network interface for exchanging data with an external device via a wireless network. In some implementations, the interface system includes an input/output device configured to receive user inputs and provide information to users. In some implementations, the input/output device includes: a display device, a light emitting diode, a speaker, a touch sensitive input device, a button, a haptic device, or any combinations thereof.

In some implementations of any of the smart inhaler systems disclosed herein, the control system is further configured to perform one or more of the following operations when the information received from the breathprint sensor system indicates that the person's identity is not verified: sending a notice to another person notifying that an unverified person attempted to use the inhaler; deactivating the inhaler apparatus; prompting the person to provide a breath sample to the breathprint sensor system; and prompting the person to provide an alternative information that is not derived from a breath sample to verify the person's identity.

In some implementations of any of the smart inhaler systems disclosed herein, the control system includes one or more processors communicatively coupled with the breathprint sensor system and the inhaler apparatus. At least one of the processors is configured to: analyze information derived from a breath sample produced by the person and determine the person has a breathing problem, associate the breathing problem with one or more environmental conditions, associate the one or more environmental conditions with location data, and create a map of the one or more environmental conditions associated with the breathing problem.

In some implementations of any of the smart inhaler systems disclosed herein, the breathprint sensor system includes: one or more first sensors having first response characteristics to compounds in gases, the one or more first sensors configured to output sensor data representing the first response characteristics; and one or more second sensors having second response characteristics to compounds in gases, the one or more second sensors configured to output supplemental sensor data representing the second response characteristics. The first response characteristics are tuned for verifying the identity of the person, and the second response characteristics are tuned for one or more biological markers. In some implementations, the one or more biological markers relate to pharmacokinetics of a drug. At least one of the processors of the control system is configured to: determine an efficacy of a dose of the drug delivered by the inhaler apparatus using the supplemental sensor data representing the second response characteristics tuned for the one or more biological markers, and determine a delivery plan of the drug based on the efficacy.

One aspect of the disclosure involves a method for verifying the identity of a person using a breathprint sensor system. The breathprint sensor system includes one or more first sensors having first response characteristics to compounds in gases. The method involves: receiving a set of test data provided by the one or more first sensors based on an exposure of the one or more first sensors to gases produced by the person; and determining whether the set of test data verifies the identity of the person. In some implementations, the breathprint sensor system further includes one or more second sensors having second response characteristics to compounds in gases. The method further involves: receiving a set of supplemental sensor data provided by the one or more second sensors based on an exposure of the one or more second sensors to the gases produced by the person; identifying a biological or environmental condition associated with the set of supplemental sensor data; and providing information indicating the biological or environmental condition.

In some implementations of the method for verifying the identity of the person, determining whether the set of test data verifies the identity of the person involves: pre-processing the test data; and using a pattern classifier to classify the pre-processed test data into one of a plurality of classes including: (i) identity verified when a pattern of the test data is recognized as belonging to the person, and (ii) identity not verified when a pattern of the test data is recognized as not belonging to the person.

In some implementations, the method further involves, before classifying the pre-processed test data, training the pattern classifier from one or more sets of training data. In some implementations, training the pattern classifier involves: receiving one or more sets of positive training data provided by the one or more first sensors based on one or more exposures of the one or more first sensors to gases produced by the person during a training phase; providing the one or more sets of positive training data to the pattern classifier; and informing the pattern classifier that the one or more sets of positive training data belong to the person.

In some implementations of the method described above, training the pattern classifier further involves: receiving one or more sets of negative training data provided by the one or more first sensors based on one or more exposures of the one or more first sensors to gases not produced by the person during a training phase; providing the one or more sets of negative training data to the pattern classifier; and informing the pattern classifier that the one or more sets of negative training data do not belong to the person.

Another aspect of the disclosure involves a method for controlling a smart inhaler system including a breathprint sensor system, an inhaler apparatus, and one or more processors in communication with the breathprint sensor system and with the inhaler apparatus. The method involves: receiving information from the breathprint sensor system indicating whether a person's identity is verified using gases produced by the person; and controlling operation of the inhaler apparatus according to the received information. In some implementations, controlling the operation of the inhaler apparatus according to the received information involves controlling a delivery of a drug according to the received information.

In some implementations, controlling the operation of the inhaler apparatus according to the received information involves performing one or more of the following operations when the information received from the breathprint sensor system indicates that the person's identity is not verified: sending a notice to another person notifying that an unverified person attempted to use the inhaler; deactivating the inhaler apparatus; prompting the person to provide a breath sample to the breathprint sensor system; and prompting the person to provide an alternative information that is not derived from a breath sample to verify the person's identity.

Some or all of the methods described herein may be performed by one or more devices according to instructions (e.g., software) stored on non-transitory media. Such non-transitory media may include memory devices such as those described herein, including but not limited to random access memory (RAM) devices, read-only memory (ROM) devices, etc. Accordingly, other aspects of the subject matter described in this disclosure can be implemented in a non-transitory medium having software stored thereon. One aspect of the disclosure provides a non-transitory computer-readable medium storing computer-readable program code to be executed by one or more processors, the program code including instructions to cause a breathprint sensor system including one or more first sensors having first response characteristics to compounds in gases to: receive a set of test data provided by the one or more first sensors based on an exposure of the one or more first sensors to gases produced by a person; and determine whether the set of test data verifies an identity of the person.

In some implementations, determining whether the set of test data verifies the identity of the person involves: pre-processing the test data; and using a pattern classifier to classify the pre-processed test data into one of a plurality of classes including: (i) identity verified when a pattern of the test data is recognized as belonging to the person, and (ii) identity not verified when a pattern of the test data is recognized as not belonging to the person.

In some implementations, the computer-readable program code further includes instructions to cause the breathprint sensor system to: receive a set of supplemental sensor data provided by one or more second sensors based on an exposure of the one or more second sensors to the gases produced by the person; identify a biological or environmental condition associated with the set of supplemental sensor data; and provide information indicating the biological or environmental condition, where the breathprint sensor system further includes the one or more second sensors having second response characteristics to compounds in gases.

One aspect of the disclosure provides a non-transitory computer-readable medium storing computer-readable program code to be executed by one or more processors, the program code including instructions configured to cause a smart inhaler system including a breathprint sensor system and an inhaler apparatus to: receive information from the breathprint sensor system indicating whether a person's identity is verified using gases produced by the person; and control operation of the inhaler apparatus according to the received information.

Details of one or more implementations of the subject matter described in this specification are set forth in the accompanying drawings and the description below. Other features, aspects, and advantages will become apparent from the description, the drawings, and the claims. Note that the relative dimensions of the following figures may not be drawn to scale.

### BRIEF DESCRIPTION OF THE DRAWINGS

Details of one or more implementations of the subject matter described in this specification are set forth in the accompanying drawings and the description below. Other features, aspects, and advantages will become apparent from the description, the drawings, and the claims. Note that the relative dimensions of the following figures may not be drawn to scale. Like reference numbers and designations in the various drawings indicate like elements.
Figure 1A is a block diagram that shows an example of components of a smart inhaler system in which some aspects of the present disclosure may be implemented.
Figure 1B is a block diagram that shows an example of components of a breathprint sensor system in which some aspects of the present disclosure may be implemented.
Figure 2 is a flow diagram that outlines one example of a method for verifying a user's identity using the user's breathprint.
Figure 3 shows a flow diagram that outlines an example of a method of operating a smart inhaler system for verifying a user's identity using a breathprint sensor array.
Figure 4 is a block diagram that shows an example of a system for verifying a user's identity based on a pattern classification of data from a sensor array.
Figure 5 is a schematic diagram of a neural network configured to perform pattern classification for verifying a user's identity.
Figure 6A is a diagram illustrating an example of a pattern of response of a sensor array in a breathprint sensor system.
Figure 6B is a diagram illustrating an example of a pattern of response of two sensor arrays in a breathprint sensor system.
Figure 7 is a block diagram that shows examples of components of a system in which some aspects of the present disclosure may be implemented through a computer network.

### DETAILED DESCRIPTION

One way of administering drugs to people is through inhalation. In some implementations, an inhaler can be inserted into the person's mouth, which is an environment specific to the person using the inhaler. In some other implementations, an electronic nose apparatus can be used to identify persons or confirm that a specific person is administering a drug.

Conventional smart inhalers are used to deliver drugs such as asthma or chronic heart failure (CHF) medication by inhalation, as well as other drugs such as an inhalable powder version of insulin. Conventional smart inhalers mainly address compliance and adherence problems. For instance, conventional smart inhalers provide functions to ensure the right dose, right form, and right drug are administered at the right time.

Some of the disclosed implementations are configured to facilitate identification of the "right patient" in convenient ways for a user, such as a health care professional or a patient. Identity verification can be helpful in connection with verifying drug compliance and adherence, determining efficacy and safety of the drug, and enabling a pay-per-outcome model. Easy-to-use positive verification of a patient's identity is valuable especially in the case of smart wireless mobile drug delivery mechanisms. It is also helpful in remote mobile drug trials. For instance, it can be desirable to deliver inhalable drugs to multiple patients in a hospital when a drug delivery system can verify and keep track of the identities of patients receiving the drug. In another example, it can be helpful for a doctor to remotely monitor an out-patient's inhalable drug administration using an automated system that can verify and keep track of the identities of patient.

Some of the disclosed implementations provide a system for delivering drugs by inhalation and verifying the patient's identity in a user-transparent manner. Some of the disclosed implementations are configured to determine if a user has properly delivered and consumed a drug by analyzing the breath exhaled by the user after the user has administered and inhaled the drug. Some of the disclosed implementations provide a smart inhaler operable to detect biomarkers of medical conditions by analyzing the air exhaled by the user. Some of the disclosed implementations analyze compounds, e.g., volatile organic compounds (VOCs) contained in exhaled air, thereby determining a breathprint indicative of the identity of the user, the effective administration of drugs, and/or the biomarker for medical conditions. Some of the disclosed implementations integrate a fingerprint sensor (e.g., on a button to be pressed by the user) or a DNA analyzer (e.g., to analyze DNA in saliva) into a smart inhaler for user identification verification.

Some of the disclosed implementations integrate into a smart inhaler a sensor that can be trained to recognize a person-specific signature from the breath of the person, generally referred to herein as a breathprint of the person. The signature is generally in the form of a pattern of information associated with a person, where the pattern is derived from analyses of compounds contained in the breath of the person. Although a person is described in this disclosure as the provider of breath samples that are analyzed to verify the identity of the person, it is understood that the breath of an animal other than a human (e.g., a mammal, a bird, or a reptile) can also be used to verify the identity of the animal in a veterinary setting using implementations disclosed herein.

Some implementations of the disclosure provide breathprint sensor systems that can obtain breathprints that are specific to different individuals. Some implementations of breathprint sensor systems can obtain breathprints that persist over time. Some implementations provide breathprint sensor systems that are cost effective to produce. Some implementations are suitable for applications in a mobile platform.

The breathprint can be analyzed in various ways. For example, sudden changes in breathprint may signal changes in the medical condition of a patient. The gases in a person's breath are a vaporized biofluid, like urine, containing metabolic phenotypes in a unique pattern for that person. Thus, in some implementations, an electronic sensor array such as an "electronic nose" can be incorporated to recognize VOCs in a person's breath.

The biofluid of a person's breath is rich in VOCs. Research using mass spectrometry has identified breathprints characterized by m/z (molecular mass to charge ratio) of ionized compounds in breath samples, e.g., m/z = 59 for acetone. Sinues, et al. (2013), Human Breath Analysis May Support the Existence of Individual Metabolic Phenotypes, PLOS One. However, mass spectrometry has not been implemented in a mobile drug delivery system due to size and weight problems of mass spectrometry equipment. Some of the disclosed implementations provide an electronic nose integrated into a mobile personal smart inhaler system. The electronic nose can be used to recognize a particular user by analyzing the gases exhaled or otherwise provided by the user.

In some implementations, an example of an electronic nose system utilizes an array of non-specific sensors to detect a "fingerprint" response to a person's breath. Pattern classification and/or recognition algorithms are applied to verify the identity of the person. The input breath induces a reversible physical change in the sensing material which in turn causes a change in electrical properties of the sensor elements (or "cells"), such as their electrical conductivity. These changes are transduced into electrical signals that are then processed prior to pattern recognition.

In some implementations, an electronic nose includes an array of sensors, where each sensor has a polymer layer having a variable conductance based on exposure to VOCs in gases. In some implementations, the sensors have different response characteristics to various compounds such as VOCs. Upon exposure to the vapor, the conductance of the polymer in each sensor changes in a different fashion than in other sensors in the array. The patterns of the conductance of the sensors may be associated with different types of objects or conditions of an object.

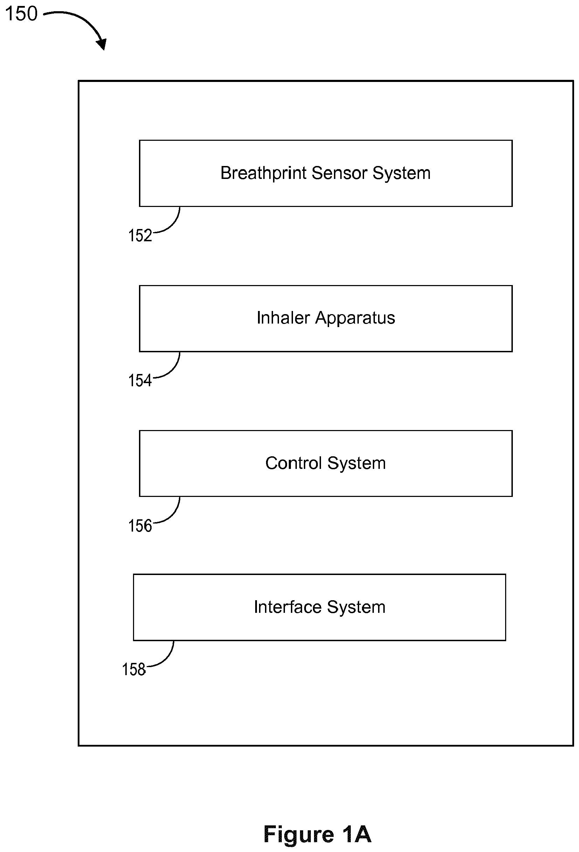

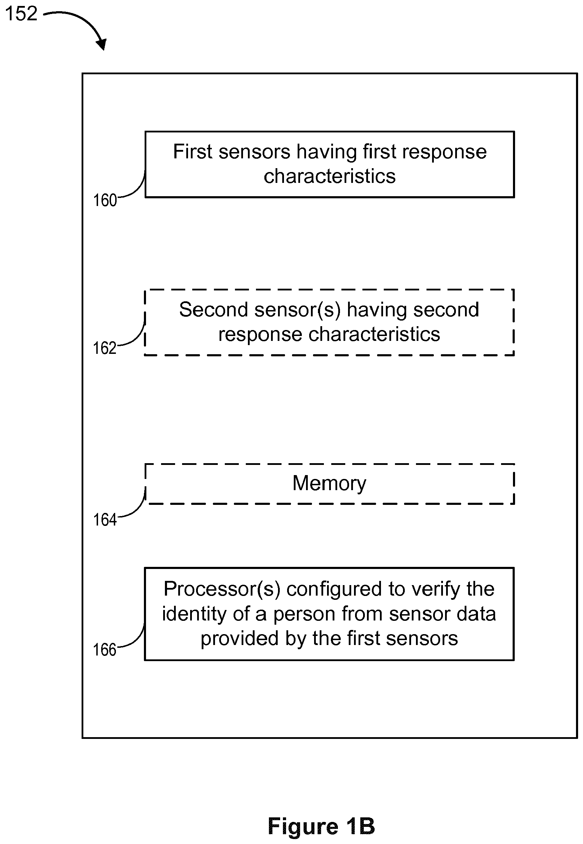

Figure 1A is a block diagram that shows an example of components of a smart inhaler system in which some aspects of the present disclosure may be implemented. As illustrated in the example in Figure 1A, the smart inhaler system includes a breathprint sensor system 152, an inhaler apparatus 154, a control system 156, and an interface system 158. In some implementations, the breathprint sensor system 152 may be implemented according to the description associated with Figure 1B below. The inhaler apparatus 154 is configured to deliver drugs to a user when it is received by the mouth or nostrils of the user.

The control system 156 is configured to control the operation of the smart inhaler system. In one example, the control system 156 is configured to control a delivery of a drug by the inhaler apparatus 154 based on information provided by the breathprint sensor system 152 or by the interface system 158. In various implementations, the control system 156 is configured to control the quantity, timing, and other characteristics of the drug.

In some implementations, the control system 156 includes at least one of a general purpose single- or multi-chip processor, a digital signal processor (DSP), an application specific integrated circuit (ASIC), a field programmable gate array (FPGA) or other programmable logic device, discrete gate or transistor logic, or discrete hardware components. In some implementations, one or more processors of the control system 156 are communicatively coupled with the breathprint sensor system and/or with the inhaler apparatus. In some implementations, the one or more processors are configured to provide instructions to control the breathprint sensor system and/or the inhaler apparatus. In some implementations, one or more processors are disposed in a housing that contains or is attached to the breathprint sensor system and/or the inhaler apparatus. In some implementations, at least one of the processors is configured to communicate through a computer network with the breathprint sensor system and/or with the inhaler apparatus. In some implementations, the control system includes two or more processors distributed over a computer network.

The interface system 158 includes an interface configured to input data to and/or output data from the control system 156, the inhaler apparatus 154, and/or the breathprint sensor system 152. In some implementations, the interface system 158 communicatively connects these elements to a memory (not shown in this figure) and/or an external device (e.g., through a wired or wireless port). In some implementations of the disclosure, the interface system 158 is optional.

In some implementations, the interface system 158 includes a wireless network interface for remotely communicating with an external device, either directly or through a computer network such as an intranet or the Internet. For instance, in some implementations, the smart inhaler system can communicate through the interface system with an application running on a mobile device communicatively linked to the smart inhaler system. In various implementations, the external device may be a diagnostic device, a physiological sensing device, an environmental sensing device, a smart watch, or a wireless phone. In some implementations, the interface allows for simultaneous communication with multiple external devices. In various implementations, the external device communicates dose information to the smart inhaler based on data from the breathprint sensor system, the inhaler apparatus, and/or the control system. In some implementations, the external device communicates dose information to the smart inhaler without requiring any user input.

In some implementations, the interface system 158 includes an input/output device configured to provide information to users and receive user inputs. For example, the input/output device includes one or more of the following: a display device, a light emitting diode, a speaker, a touch sensitive input device, a button, and a haptic device. In some implementations, the input/output device is capable of outputting information about the identity of a person determined by the breathprint sensor system 152. In some implementations, the input/output device is capable of outputting information about delivery of a drug (e.g., recommended dosage or time for administering the drug) based on information provided by the breathprint sensor system 152. For instance, the control system 156 can receive information from biological sensors of the breathprint sensor system indicative of a biological condition of the user. In response, the control system 156 may control the input/output device to output a recommendation about a dose of a drug based on the biological condition. For instance, if the control system determines that a biomarker, e.g., NO (Nitric Oxide), exists in the breath of the user indicative of a severe level of asthma, the smart inhaler system may provide a recommended dose of an asthma drug through the input/output device. In various embodiments, the input/output device may output the recommended dose (or other information) to a user in visual, auditory, haptic, olfactory, or gustatory forms. In some implementations, the control system 156 can determine an environmental condition from sensor data provided by environmental sensors, and the input/output device is further configured to provide warnings about environmental risks, such as an elevated level of allergens or pollutants.

In some implementations, the smart inhaler system includes a storage medium configured to store data representing operations of the breathprint sensor system or components thereof. In these implementations, the smart inhaler system can record information at different times, such as patient identity, time of drug administration, and drug dosage. In some implementations, such stored information may be analyzed and/or used to control the inhaler. For instance, the inhaler may adjust its dosage based on information regarding previous drug administration, biological conditions, or environmental conditions.

Figure 1B is a block diagram that shows an example of components of a breathprint sensor system 152, in which some aspects of the present disclosure may be implemented. The breathprint sensor system 152 can be implemented in the smart inhaler system 150 as illustrated in Figure 1A. In this example, the breathprint sensor system 152 includes a plurality of first sensors 160 having first response characteristics to compounds in gases. The first sensors 160 are configured to output sensor data representing the first response characteristics. In this example, the breathprint sensor system 152 also includes one or more second sensors 162 as further described below. The dash line of block 162 in Figure 1B indicates that the second sensors are optional for some implementations of breathprint sensor systems. The second sensors 162 have second response characteristics different from the first response characteristics of the first sensors 160. The second sensors 162 are configured to output sensor data representing the second response characteristics. The breathprint sensor system 152 as shown here also includes a memory 164 configured to store sensor data. The dash line of block 164 indicates that the memory 164 is optional in some implementations of a breathprint sensor system. In some implementations, a memory can be external to the breathprint sensor system. For instance, a smart inhaler system may include a memory and the breathprint sensor system. While the breathprint sensor system does not have a built-in memory, the memory of the smart inhaler system may store information obtained from the breathprint sensors. In some implementations, a networked storage may store information for the breathprint sensor system.

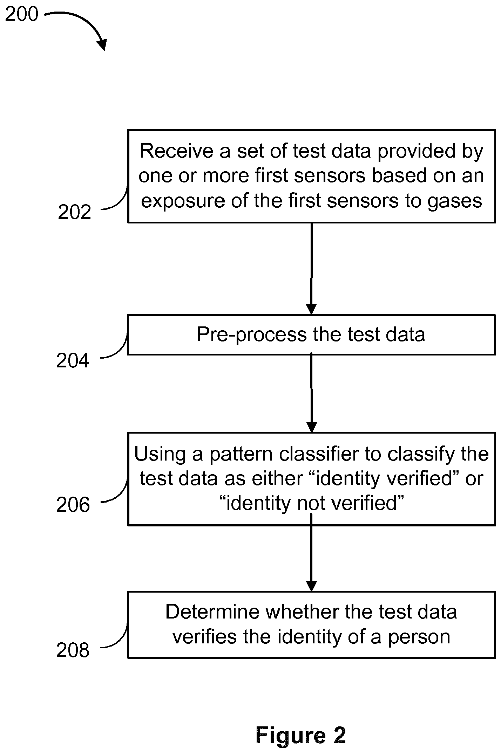

As shown in the example in Figure 1B, the breathprint sensor system 152 includes one or more processors 166. The processors are in communication with the first sensors 160. In some implementations including the second sensors 162 or the memory 164, the processors are in communications with the second sensors 162 and the memory 164. The one or more processors 166 are configured to verify the identity of a person from sensor data provided by the first sensors 160. Figure 2 illustrates an example of a process that the processors 166 may implement to determine the identity of the person.

In some implementations, an electronic nose system is used as the sensors for the breathprint sensor system 152. In some implementations, an electronic nose system includes an array of non-specific sensors, and hardware and software for implementing digital signal processing and pattern classification algorithms. The electronic nose system is used in classifying or recognizing a compound without having to break the compound into its components. One or more gases are detected by the electronic nose system. The gases may be from the mouth or the nostrils of a user. Some compounds in the gases originate from the body of the user, providing biometrics of the user.

In some implementations, the first sensors 160 of breathprint sensor system 152 are configured to output sensor data representing the first response characteristics. In some implementations, the sensors have different response characteristics to volatile organic compounds in gases. In some implementations, the first sensors are tuned to provide different response characteristics from one person to another person.

Various sensor materials may be employed in electronic nose systems, including but not limited to conducting polymer, conducting polymer composites, intrinsically conducting polymers, metal oxides, graphene, and other materials. The sensor materials may provide a mechanism for detecting a charge transfer between the sensor and molecules of a compound of interest.

Under various circumstances, it may be desirable to provide sensors configured to operate in certain temperatures. For instance, some conventional sensors such as metal oxide sensors have acceptable dynamic sensitivity at relatively high temperatures, e.g. at higher than about 200°C. These conventional sensors may only perform well at high temperatures. Operating a breathprint sensory system with such conventional sensors may require additional energy and hardware for heating the sensors and/or the gases. Therefore, in some implementations, the disclosed sensors are configured to obtain sensor data when a temperature at a surface of one or more of the sensors, i.e., a sensor surface temperature, is no higher than about 200°C, or no higher than about 100°C.

Under some circumstances, implementations of the disclosed sensors are configured to obtain sensor data when the sensor surface temperature is in a range consistent with common operating temperatures of environments in which users use the breathprint sensors. For example, some implementations of the disclosed sensors are configured to obtain sensor data with a sensor surface temperature from about 0 °C to about 50 °C. In some other implementations, the sensor surface temperature is in a wider temperature range to accommodate more extreme sensing environments such as outer space, near the Earth's poles, or in underground environments. Thus, some implementations of the disclosed sensors are configured to obtain sensor data with a sensor surface temperature from about -50 °C to about 100 °C, or from about -100 °C to about 200 °C.

In some implementations, the sensors used in a breathprint system are configured to obtain sensor data with an unmodulated sensor surface temperature. For instance, an unmodulated surface temperature may be a constant or substantially constant surface temperature, such as a temperature that varies no more than about 10°C. In another example, an unmodulated surface temperature is a temperature which passively varies as a result of the ambient temperature, often in a gradual manner.

In some implementations involving an electronic nose system, conducting polymer composites are used as sensing materials of sensors. The conducting polymer composites include conducting particles, e.g., polypyrrole and carbon black, interspersed in an insulating polymer. On exposure to gases, gas permeates into the polymer inducing the polymer to expand. This induced expansion of the polymer composite causes an increase in resistance of the polymer composite as expansion reduces number of conducting pathways for charge carriers. In some implementations, conducting polymer composites have less than 20 second response time, or more preferably less than 10 second response time, or more preferably 2-4 second response times.

In some implementations, conducting polymer sensors can be made from conducting polymer composites that are inexpensive or easy to manufacture, which is suitable for breathprint sensors in disposable smart inhalers. In some implementations, conducting polymer sensors can provide data for detecting breathprints under one or more of the following conditions: small amount of vapor, dilution by ambient air, changing environments (e.g., temperature, light, and air flow), low compound concentration, or high sensing speed.

The mouth or the nose of a person provides a stable operating environment for an electronic nose in terms of temperature and humidity, which environment can provide good response conditions for conducting polymer sensors. In some applications, conducting polymer sensors are implemented in disposable devices having limited time of use. The limited time of use makes the application of conducting polymer sensors practical even when the polymer materials age relatively quickly.

The breathprint sensor system 152 of Figure 1B may optionally include one or more second sensors 162 having response characteristics different from the response characteristics of the first sensors 160. The second sensors are also referred to as augmented sensors herein after. These second sensors 162 are provided to detect biological or environmental conditions. In some implementations, these second sensors 162 are tuned to determine one or more biological or environmental conditions. For instance, the second sensors 162 may be tuned to provide data for determining a nitric oxide (NO) level in a user's breath, which can be used to determine the severity of asthma. In another example, the second sensors 162 can be tuned to provide data to determine allergens, pollutants, and other health related factors in the air.

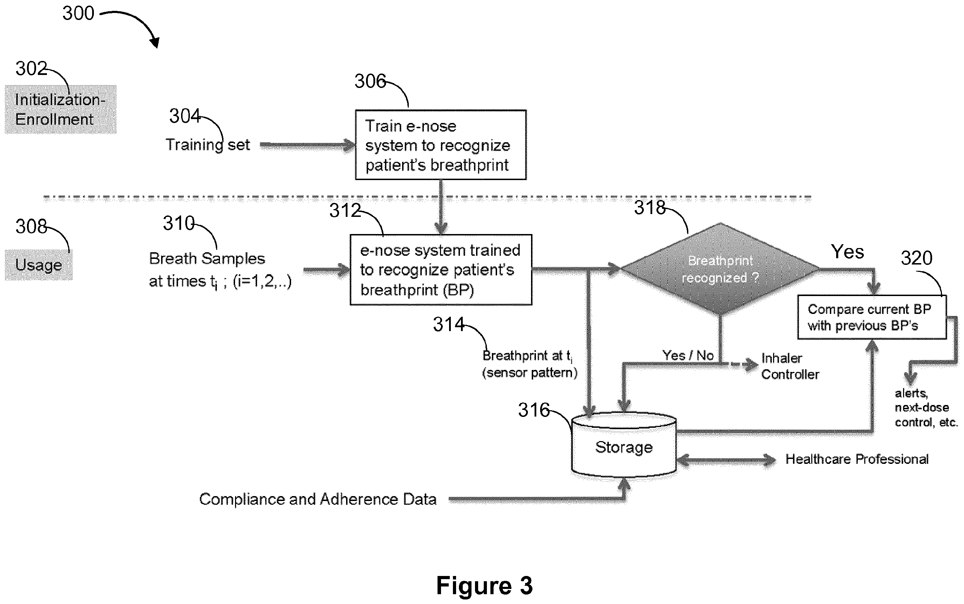

Figure 2 is a flow diagram that outlines one example of a method 200 for verifying a user's identity using the user's breath. One aspect of the disclosure may implement this method by executing instructions on the one or more processors 166 of the breathprint sensor system 152 described above in connection with Figure 1B. As illustrated, method 200 involves receiving a set of test data provided by one or more first sensors based on an exposure of the first sensors to gases during a test phase. See block 202. In various applications, the gases during a test phase are provided by a person using a breathprint sensor system to verify her/his identity. In some implementations, the breathprint sensor system can be used a part of a smart inhaler system. In some implementations, as shown in this figure, method 200 optionally involves pre-processing sensor data provided the by the first sensors (e.g., analog-to-digital conversion of the sensor data or filtering of the sensor data). See block 204. The sensor data may be test data provided during a test phase when a person uses the breathprint sensor system to verify her/his identity. In some implementations, method 200 optionally involves using a pattern classifier to classify the test data into one of a plurality of classes including: (i) identity verified when a pattern of the test data is recognized as belonging to the person, or (ii) identity not verified when a pattern of the test data is recognized as not belonging to the person. See block 206. In some implementations, more than two classes may be implemented, such as a third class of "no-call," when the pattern of the test data is between the first and second classes according to the classifier. In some implementations, method 200 proceeds to determine that the test data verifies the identity of the person if the pattern classifier classifies the test data as class (i). See block 208. In some implementations, the pattern classifier is a neural network pattern classifier. An applicable neural network pattern classifier is further described below with reference to Figure 3.

In some implementations, the pattern classifier is trained using one or more sets of training data. In some implementations, training of the pattern classifier occurs during a training phase before a test phase. In some implementations, training of the pattern classifier involves: receiving one or more sets of positive training data provided by the first sensors based on one or more exposures of the first sensors to gases exhaled or otherwise provided by a person during a training phase. In some implementations, the gases are provided by the person exhaling gases into an apparatus that is configured to receive gases in breath samples. In various implementations, the gases may be received by an inhaler apparatus, an over the mouth or nose mask, an apparatus configured for insertion into a body cavity, etc. The person's identity is to be tested during a test phase after the pattern classifier is trained. The training of the pattern classifier further involves: providing the one or more sets of positive training data to the pattern classifier; and informing the pattern classifier that the one or more sets of positive training data belong to the person.

In some implementations, training the pattern classifier involves: receiving one or more sets of negative training data provided by the first sensors based on one or more exposures of the first sensors to gases not provided by the person during a training phase; providing the one or more sets of negative training data to the pattern classifier; and informing the pattern classifier that the one or more sets of positive training data do not belong to the person. In some implementations, the negative training data may be derived from ambient air, gases exhaled by control subjects, or other gases not obtained from the person.

In some implementations using feature extraction techniques known in the art, the breathprint sensor system can derive a test feature vector from test data, a target feature vector from positive training data, and a control feature vector from negative training data. In some implementations, the breathprint sensor system can compare a test feature vector to a target feature vector; and based on the comparison, determine whether or not the test data verifies the identity of the person. In some implementations, comparing the test feature vector to the target feature vector involves determining a difference, or distance, between the test feature vector and the target feature vector. In some implementations, if the difference is smaller than a criterion, the method proceeds to determine that the set of sensor data identifies the person based on the comparison. In some implementations, the method can compare the test feature vector to both a target feature vector and a control feature vector, the target feature vector being derived from positive training data, and the control feature vector being derived from negative training data. In some implementations, the method determines the test data verifies the identity of the person if the test feature vector is more similar to the target feature vector than to the control feature vector.

According to some implementations, method 200 may be implemented in a non-transitory medium having software stored thereon. In some implementations, method 200 may be performed, at least in part, by one or more processors of a sensor system (e.g., by processors 166 shown in Figure 1B). In some implementations, method 200 may be performed by one or more processors in the smart inhaler system 150 in Figure 1A.

Returning to Figure 1A, some implementations of the disclosure provide a smart inhaler system 150 including a control system 156. The control system 156 is configured to receive information provided by the breathprint sensor system 152. The received information indicates whether a person's identity is verified. In some implementations, the control system 156 may include one or more processors that can analyze information from a breathprint sensor system to determine if the person's identity is verified. In various implementations, if the person's identity is verified, the control system 156 controls delivery of a drug through the inhaler apparatus 154, such as by controlling the dosage, concentration, timing and other aspects of the drug. In some implementations, the control system is configured to perform one or more of the following operations if the identity of the person providing a breath sample to the smart inhaler is not verified: sending a notice to another person (such as a doctor or a caregiver) or to a computer system to notify that an unverified person attempted to use the smart inhaler; deactivating the inhaler apparatus; prompting the person to provide an additional breath sample to the breathprint sensor system; or prompting the person to provide alternative information to verify the person's identity (e.g., by entering a secured password). In some implementations, the smart inhaler system may also include a fingerprint sensor, through which the person may provide a fingerprint sample as the alternative information to verify his or her identity. In some implementations, the control system 156 stores compliance data in a storage, including results of identity verification, dosage, and timing of administration of the dosage by the inhaler, as described below with reference to Figure 3.

In some implementations, the smart inhaler system 150 has one or more processors as described above. In some implementations, at least one of the processors is configured to analyze information derived from a breath sample of a person and determine the person has a breathing problem. This determination may be based on data provided by one or more sensors of the breathprint sensor system 152. The smart inhaler system 150 can monitor the person's breathing indices (e.g., rate of inhalation or inhalation volume) to determine when the person is having breathing problems (e.g., labored breathing or asthma attacks). In some implementations, at least one of the processors is further configured to associate a breathing problem with one or more environmental conditions. In some implementations, at least one of the processors is further configured to associate the one or more environmental conditions with location data and create a map of the environmental conditions associated with the breathing problem.

In some implementations, the smart inhaler system 150 is implemented as a part of a system that includes a database with information about the usage of multiple persons. At least one of the processors of the smart inhaler system 150 is configured to aggregate information derived from the multiple persons to create a map of environmental conditions associated with breathing problems.

In some implementations, the smart inhaler system 150 includes a breathprint sensor system including: one or more first sensors having first response characteristics to compounds in gases, and one or more second sensors having second response characteristics to compounds in gases. The first sensors are configured to output sensor data representing the first response characteristics; and the second sensors are configured to output supplemental sensor data representing the second response characteristics. In some implementations, the first response characteristics are tuned for verifying the identity of the person, and the second response characteristics are tuned for one or more biological markers. In some implementations, the one or more biological markers relate to pharmacokinetics of a drug. In some implementations, the smart inhaler system test biological markers in a person's breath, which biological markers are specific to a drug being administered, to verify that the user is in fact taking the right drug. As described elsewhere herein, the smart inhaler system can use one or more processors to test data provided by supplemental sensors of a breathprint sensor system to test the biological markers.

In some implementations, the smart inhaler system records time and/or dose of information of a drug delivery and stores the information in a database. In some implementations, using one or more processors, the smart inhaler system compares time and/or dose information of a delivery to a schedule stored in the database to determine compliance information. In some implementations, the smart inhaler system controls an inhaler apparatus according to the compliance information.

In some implementations, at least one processor of the smart inhaler system 150 is configured to determine an efficacy of a dose of the drug delivered by an inhaler apparatus using the supplemental data representing the second response characteristics tuned for the one or more biological markers. In some implementations, at least one processor of the smart inhaler system is further configured to determine a delivery plan of the drug based on the efficacy determined by the processors. For example, the processor may determine to increase or decrease the dosage of the drug if compounds in the patient's breath indicate pharmacokinetic parameters that warrant a change in dosage.

In some implementations, at least one processor of the smart inhaler system is configured to compare efficacy data of multiple persons, and recognize patterns of efficacy data across a population. In these implementations, the efficacy data of the population can be stored in a database accessible by at least one processor of the smart inhaler system.

In some implementations, the smart inhaler system 150 may be used to determine if a person (e.g., a pilot) has consumed a drug (e.g., alcohol) and/or if the person is in a physiological state fit for performing certain activities (e.g., operating a plane). In some implementations, using sensor data from first sensors as described elsewhere herein, the smart inhaler system 150 may verify the identity of a person (e.g., an airline pilot) is using the device to test drug consumption. At least one processor of the smart inhaler system is configured to determine whether the person has taken a drug using supplemental sensor data provided by second sensors of a breathprint sensor system 152. The supplemental sensor data represents second response characteristics tuned for the one or more biological markers. At least one processor of the smart inhaler system is further configured to determine whether the person is in a physiological state fit for performing an activity based the supplemental data indicating levels of the biological markers. For instance, the system may determine whether a pilot has taken too much alcohol to safely operate a plane.

In some implementations, using smart inhaler system 150, one may monitor persons with drug addictions. In such applications, the smart inhaler system can 1) verifies the identity of the person using the first sensors, 2) monitors that the person has not used a drug using the second sensors, and/or 3) send the test results to an authority or agency.

Figure 3 shows a flow diagram that outlines an example of a method 300 for operating a smart inhaler system for determining a person's identity using a breathprint sensor array. During the initialization or enrollment stage (block 302), the system receives a training set data 304 and uses it to train an electronic nose that can recognize or classify the patient's breathprint (block 306). The training set data 304 is obtained by an electronic nose system, such as the breathprint sensor system 152 described in Figure 1B. In some implementations, the training set includes positive training data obtained from the exhaled air of a person to be identified by the breathprint sensor system. In some implementations, the training set also includes negative training data not obtained from the exhaled air of the person (e.g., data from ambient air or control subjects). In some implementations, at this enrollment stage, the breathprint sensor system extracts one or more target feature vectors from the data of the person. In some implementations involving negative training, the system can also extract one or more control feature vectors from data not obtained from the person. The extracted feature vectors or other information derived from training data are provided to a pattern classifier to train the pattern classifier to classify breath samples as (1) identity verified for the person or (2) identity not verified for the person.

During the usage stage (block 308), breath samples are obtained as test data (block 310) at various times (tᵢ, i=1, 2...). Test data are then provided to an electronic nose system that has been trained to recognize the user's breathprint (block 312). In some implementations, the recognition of the user's breathprint is achieved by a pattern classifier as described herein (see block 318). In some implementations, the recognition of the user's breathprint is determined by a binary classification using data derived from breath samples: identity of the user is verified and identity of the user is not verified. In some implementations, the binary classification may be implemented by a neural network pattern classifier, such as a neural network described below. In some implementations, the binary classification may be implemented by determining the difference (or distance) between a test feature vector and a target feature vector as described above.

In the example shown in Figure 3, the test data and/or information derived therefrom at various times (314) are stored in a storage (316) of the smart inhaler system. The identity verification results of the breathprints may also be stored. Furthermore, the results may be used to control an inhaler apparatus of the smart inhaler system. For instance, in some implementations, the inhaler apparatus may not administer the drug if the user's identity is not verified. In other implementations, the inhaler may issue a warning when the user's identity is not verified.

In some implementations of the smart inhaler system, as shown in Figure 3, the smart inhaler system further compares information derived from the current breathprint sample and one or more previous breathprint samples stored in the memory (block 320). The previous breathprint samples may be obtained from the same user, or from other users. Based on results of the comparison, in various implementations, the smart inhaler system may issue alerts (e.g., when detecting a similarity between the particular user and other users having a health risk or a change of a biomarker indicative of a health risk), or instructions for a next dose (e.g., when detecting a biomarker relevant in determining an effective dosage).

Various information obtained by the smart inhaler system, including compliance and adherence data, may be stored on storage 316. The stored information may be exchanged with healthcare providers through various communication interfaces, such as a wireless network interface described herein.

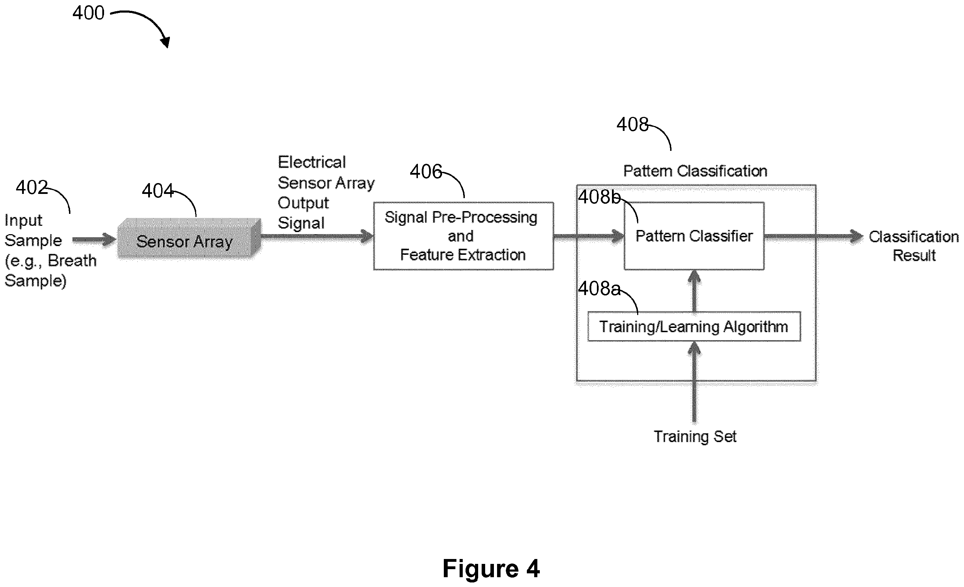

Figure 4 is a block diagram that shows an example of a breathprint sensor system 400 for verifying a user's identity based on a pattern classification of data from a sensor array 404. The sensor array 404 receives a breath sample as an input sample 402 that is to be analyzed to determine the identity of the user providing the sample. The sensor array 404 may be implemented according to the sensors described above. The sensor array provides output signals upon exposure to the breath sample. The breathprint sensor system 400 then applies pre-processing and feature extraction to the sensor output signal. See block 406. Signal pre-processing includes, e.g., digitization of sensor array output signal for pattern classification. Pre-processed data, such as digital array of values, may then undergo feature extraction, where feature vectors are extracted and provided as an input to the Pattern Classifier 408b. Various feature extraction techniques may be used to obtain feature vectors that efficiently capture and express salient characteristics of sensor data. The feature vectors obtained by feature extraction are provided as an input for pattern classification. See block 408. In some implementations, pattern classification requires training of a pattern classifier. In a training phase, training or learning algorithms 408a are used to train a pattern classifier 408b to determine whether an input has a pattern belonging to the class of "identity verified" and the class of "identity not verified." Then the classification result is provided as an output.

As explained above, training data is provided in a training phase. In some implementations, positive training data obtained from a person to be tested are used to train the pattern classifier. In some implementations negative training data not obtained from the person are provided to train the pattern classifier. After training, the pattern classifier 408b can determine whether test data or a feature vector extracted from test data should be classified as "identity verified" or "identity not verified." The classification result depends on the classifier's training, which involves data from the person and data not from the person, and knowledge of the identity of the source of the data. In some implementations, additional incremental training or tuning can be performed during a testing phase, i.e., normal use, in an unsupervised fashion.

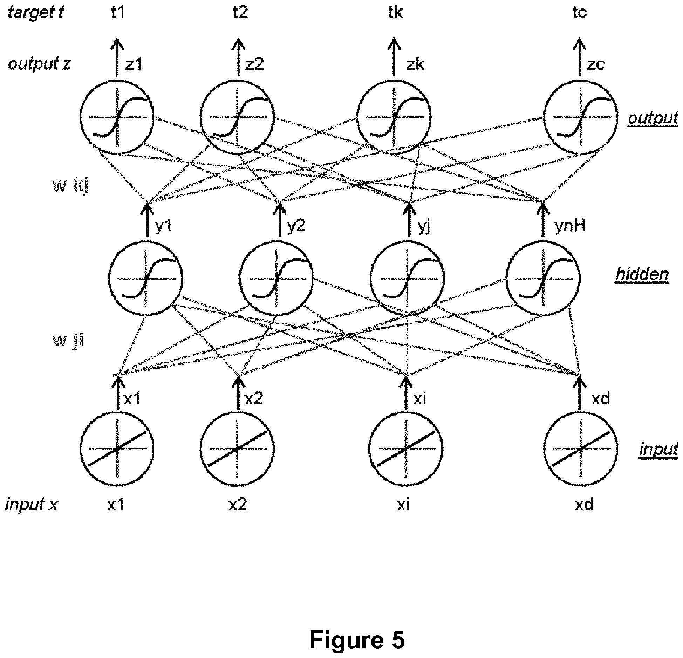

Various methods for pattern classification may be employed. Artificial Neural Networks (ANN), such as the one illustrated in Figure 5 is used in some implementations for pattern classification of 408 to determine user's identification. Figure 5 is a schematic diagram of a neural network configured to perform pattern classification for determining a user's identity. This process mimics the biological sensory process of olfactory function, in which the nose and the brain classify different smells.

As illustrated as an example in Figure 5, an ANN is a multi-layer network (e.g., Multi-Layer Perceptron network) including input and output layers plus hidden layers. Features are presented to the input layer, and the output layer expresses the classification result. The parameters of the ANN are determined by training (supervised learning) using algorithms such as Back Propagation. A 3-layer ANN is depicted in the Figure 5 as an example. The number of output nodes can be 2 in this application, one node for classification of "identity verified" indicating that an information pattern of a test sample is similar to an information pattern of one or more training samples provided by the person to be verified. In some implementations, the number of input nodes is equal to the number of sensors in the array or the number of features extracted from sensor data. One skilled in the art may implement different numbers of nodes and layers to achieve desirable performance of the ANN.

The weights of the hidden layer of the ANN are determined as a result of training, e.g., using back propagation algorithm, by presenting to the network positive and negative samples. The weights are modified iteratively to reduce classification errors. In one implementation, the patient provides his/her breath samples multiple times in a training phase, guided by, e.g., an application running on a mobile device connected to the smart inhaler. Other samples, e.g., samples of the ambient air can be used as negative training examples. This training stage is also referred to as the enrollment stage above.

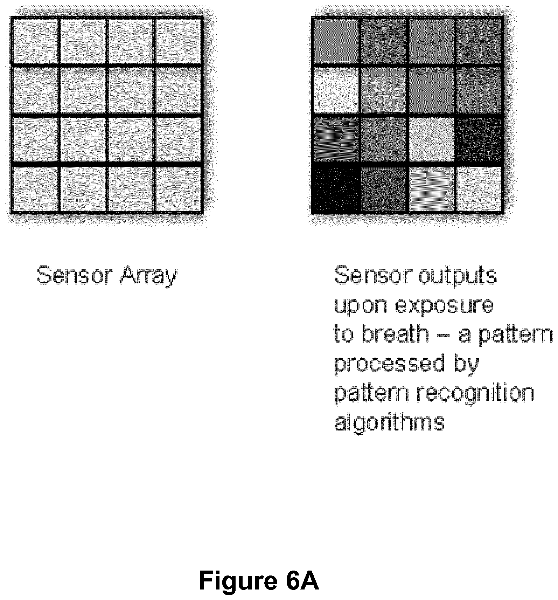

In some implementations, a breathprint sensor system includes a primary array of sensors (also referred to as first sensors) configured to determine the identity of a user. Figure 6A is a diagram illustrating an example of a pattern of response of a first sensor array in a breathprint sensor system. In this illustrative example, the sensor array has 16 sensors shown on the left 4x4 array. In some implementations, the first sensors have nonspecific response characteristics. Namely, the sensors are not specifically tuned to have different levels of response dependent on a specific property. Upon an exposure to a breath sample, the sensors produce different levels of responses as illustrated by different shades of gray in the right 4x4 array. The sensors provide data representing various response levels, which are then processed by a breathprint sensor system. In some implementations, the data are analyzed by a pattern classifier to determine the identity of the user as described above.

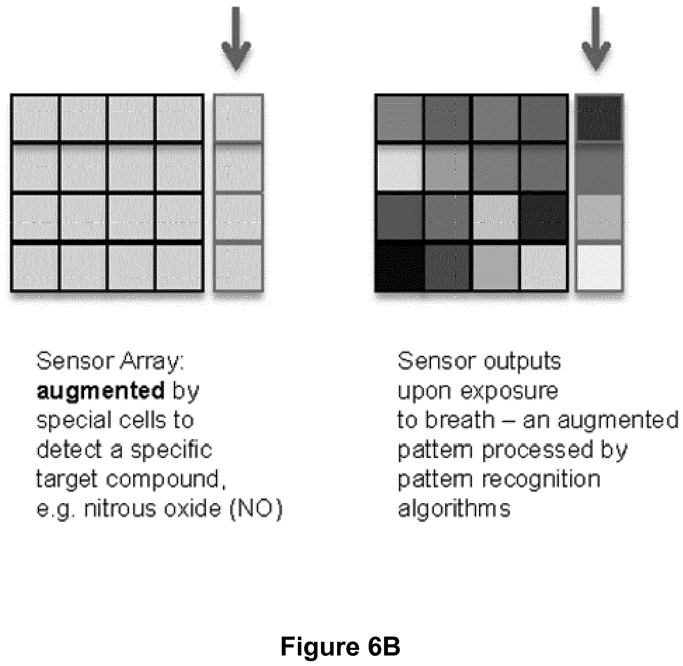

In some implementations, the breathprint sensor system also includes an augmented array of sensors (also referred to as second sensors elsewhere herein) configured to determine biological and/or environmental conditions. Figure 6B is a diagram illustrating an example of a pattern of response of a first sensor array and a second sensor array in a breathprint sensor system. As illustrated on the left half of the figure, a 4 x 4 primary sensor array is coupled with a 1 x 4 augmented sensor array. In some implementations, the augmented sensor array is specifically tuned to detect one or more target compounds, e.g., nitric oxide (NO), allergens, or pollutants. In various implementations, the augmented sensor array may be tuned to have different response characteristics to various compounds. The responses of the primary and augmented sensor arrays are illustrated on the right shown by different shades of gray. The responses of the augmented sensor array may be used to determine various biological and/or environmental conditions. It is worth noting that the illustrated sizes of the arrays do not indicate actual preferred sizes of sensor arrays for determining user identities, biological conditions, or environmental conditions.

In some implementations, augmented sensor array are built and trained *a priori* to recognize VOCs that are specific to disease related markers. For instance, the augmented sensor array may be tuned to nitric oxide (NO) indicative of severity of asthma (mild versus severe).

In some implementations of a breathprint sensor system having augmented sensors, once a delivered drug is consumed, after oxidization and other physiological reactions, the sensors system may be able to detect other conditions in the breath: e.g., a medical condition or a response to drug.

In some implementations of a breathprint sensor system having augmented sensors, the system may detect other conditions: e.g., triggers in the environment for onset of asthma attack and other medically relevant or health-related conditions. Some implementations of the breathprint sensor system may have environmental sensors that detect environmental conditions. In some implementation, the environmental sensors may sense environmental factors after the environmental factors interact with the users.

In some implementations, sensors are tuned to differentiate various properties: user identity, disease state, drug response, etc. In some implementations, tuning involves modification of hardware. For instance, sensors can have different coatings and compositions of coatings. In one example, NO sensor resistors may vary in a range that is sensitive to different concentrations of NO. In contrast, identity signature may be affected by relative concentration of N₂, CO₂, O₂, and other compounds. So an identity sensor may need to be tuned differently from an NO sensor. In various implementations, tuning may be performed by the manufacturer or by a user. In some implementations, tuning involves adjustment of software or algorithm. In some implementations, local trimming of response characteristics are applied during tuning.

Some implementations provide a breathprint sensor system having an augmented sensor array including: one or more second sensors having different response characteristics to compounds in gases, where the second sensors are tuned to differentiate among different biological conditions of an individual. At least one processor of the breathprint sensor system is configured to receive a set of supplemental data from the second sensors in response to an exposure to gases exhaled by a person during test phase. At least one processor of the breathprint sensor system is further configured to determine if the set of supplemental data is associated with a particular biological condition. In some implementations, a second sensor array can detect nitric oxide to assess a severity of asthma from a breath sample. But the second sensor array does not provide data to obtain a signature associated with the identity of the person providing the breath sample. Instead, a primary sensor array of the breathprint system can provide data to obtain a signature associated with the identity of the person.

If a person presses the button on the inhaler without actually administering the drug into the person's respiratory system, some previously available smart inhalers would record that the drug having been administered. In some implementations, a smart inhaler system disclosed here can detect the specific oral cavity environments of the user, so that it can determine that the above situation does not actually lead to drug administration to the user. Furthermore, the smart inhaler system can determine the identity of the user, providing knowledge about whether the wrong individual is attempting to use the inhaler. Moreover, some implementations of the smart inhaler system can determine if the drug has been applied and consumed. In these implementations, a second sensor array can be used to detect change of one or more markers related to the pharmacokinetics of a drug, the markers being affected by the consumption of the drug.

Furthermore, in some implementations, the smart inhaler having an augmented sensor array can recognize VOCs as disease markers (e.g. severity of asthma). The smart inhaler can determine a next dose or issue an alert based on readings of the augmented sensor array. For instance, the smart inhaler can determine an appropriate next dose: it may recommend a rescue does three hours after a first dose, based on a biological response of a person to the first dose. The biological response can be determined by the augmented sensor array. In various implementations, a smart inhaler may have one or more of the following functions:
1) patient verification from breathprint.
2) determine disease markers.
3) determine bad environmental compounds / dangerous compounds in the air that have been inhaled by the user and appear in the user's breath. For example, chemicals generally known to be bad for health (noxious fumes, etc.) can recognized by the breathprint sensor system using pattern recognition methods.
4) determine compounds that are specific triggers of breathing problems. These compounds may be inhaled and are a subset of 3) above. The smart inhaler system may have knowledge that that compounds trigger bad responses in a user. The knowledge may be learned from the user's operations of the smart inhaler. The knowledge may also be provided in a database. In some implementations, relevant data can be managed persistently (e.g. in a cloud), so that the same user with a new inhaler can still benefit from the past learning of an old inhaler. In some implementations, relevant compounds don't have to be inhaled, but may for example be ingested (food or drink) that triggers an attack. In some situations, ingesting the compounds may leave a breathprint. A person with an allergic response to food additives may suffer an asthma attack from consuming the food additive. The person might not know she has eaten "contaminated" food, but the smart inhaler as in some implementations can detect the chemical in the user's breath and provide alert and/or suggest a dose of a drug.
5) determine drug pharmacokinetics and drug efficacy. In some implementations, the smart inhaler can monitor, through breath samples, a user's absorption, retention, metabolism of a drug. It may monitor the user's drug use and asthma attacks to learn how to optimize the drug for the user (e.g., regarding dose, delivery time, etc.).
6) verification of the right drug.

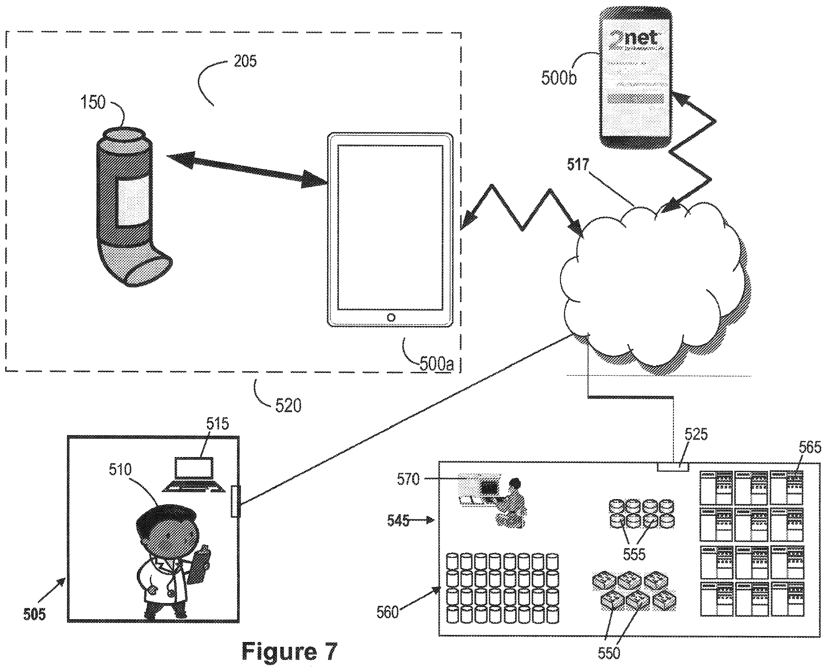

Figure 7 is a block diagram that shows examples of components of a system in which some aspects of the present disclosure may be implemented through a computer network. In some implementations, the computer network includes a wireless network component. The numbers, types, and arrangements of devices shown in Figure 7 are merely shown by way of example. In this example, various devices are capable of communication via one or more networks 517. The networks 517 may, for example, include the public switched telephone network (PSTN), the Internet and the cloud. External devices 500a and 500b shown in Figure 7 may communicate with a smart inhaler system 150. The external devices 500a and 500b, for example, may be smart phones, cellular telephones, tablet devices, etc.

At location 520, a mobile device 500a is capable of wireless communication with a smart inhaler system 150. The mobile device 500a is one example of an "external device" referenced in the foregoing discussion. The mobile device 500a may, for example, be capable of executing software to perform some of the methods described herein, such as identification functionality, determining and sending control signals to the smart inhaler system 150, receiving information from the smart inhaler system 150, or analyzing information from the smart inhaler system 150 or a data center 545.

In this example, a data center 545 includes various devices that may be configured to provide health information services via the networks 517. Accordingly, the data center 545 is capable of communication with the networks 517 via the gateway 525. Switches 550 and routers 555 may be configured to provide network connectivity for devices of the data center 545, including storage devices 560, servers 565 and workstations 570. Although only one data center 545 is shown in Figure 7, some implementations may include multiple data centers 545. In some implementations, the data center 545 may be implemented as part of an online healthcare related data service such as the 2net ™ service or Healthy Circles ™ service.

One or more types of devices in the data center 545 (or elsewhere) may be capable of executing middleware, e.g., for data management and/or device communication. Health-related information, including but not limited to information obtained by the smart inhaler system 150 and/or other information regarding authorized users of the smart inhaler system 150, may be stored on storage devices 560 and/or servers 565. Health-related software also may be stored on storage devices 560 and/or servers 565. In some implementations, some such health-related software may be available as "apps" and downloadable by authorized users.

In this example, various people and/or entities, including but not limited to health care professionals, patients, patients' families, insurance company representatives, etc., may obtain information regarding, or obtained by, the smart inhaler system 150. The information may include, but is not limited to, physiological data obtained by the smart inhaler system 150, information regarding substance delivered by the smart inhaler system 150, etc.

In some implementations, information regarding the type of substance delivered, the time of substance delivery, the dosage and/or other data may be transmitted by the smart inhaler system 150 through the network 571, and stored in one or more devices of the data center 545. Additional information, such as time stamp information, authentication information and/or location information, may be associated with substance delivery information and/or physiological data obtained by the smart inhaler system 150. In some implementations, at least some of such information may be stored on a memory disposed in a housing that also includes an inhaler apparatus. Such information may be used, for example, to create a record that substances were being administered to and/or data were being obtained from an authorized user/patient at specified times. Such information may be used to create an audit trail. In some implementations, such information may be used to enable mobile and/or remote clinical trials for drugs, instead of requiring participants in drug trials to have drugs administered only in a particular location, such as a medical research center or a healthcare facility.

In some examples, authorized people and/or entities may obtain such information via the data center 545. Alternatively, at least some people and/or entities may be authorized to obtain such information via a data feed from the smart inhaler system 150. One or more other devices (such as mobile devices 500a and 500b or devices of the data center 545) may act as intermediaries for such data feeds. Such devices may, for example, be capable of applying data filtering algorithms, executing data summary and/or analysis software, etc. In some implementations, data filtering, summary and/or analysis software may be available as "apps" and downloadable (e.g., from the data center 545) by authorized users.

A family member of an authorized user may log into the system, via the mobile device 500b, in order to access physiological data obtained by a smart inhaler system 150 from the user. Figure 7 also depicts a doctor's office 505, from which a health care professional 510 is using a laptop 515 to access information from the data center 545. The information may include information obtained by (and/or substances delivered by) the smart inhaler system 150.

The description herein is directed to certain implementations for the purposes of describing the aspects of this disclosure. However, a person having ordinary skill in the art will readily recognize that the teachings herein may be applied in a multitude of different ways. It is contemplated that the described implementations may be included in or associated with a variety of electronic devices such as, but not limited to: mobile telephones, multimedia Internet enabled cellular telephones, mobile television receivers, wireless devices, smartphones, Bluetooth® devices, personal data assistants (PDAs), wireless electronic mail receivers, hand-held or portable computers, netbooks, notebooks, smartbooks, tablets, global positioning system (GPS) receivers/navigators, cameras, camcorders, wrist watches, electronic reading devices (e.g., e-readers), mobile health devices, etc. The teachings herein also may be used in applications such as, but not limited to, electronic switching devices, radio frequency filters, sensors, including but not limited to biometric sensors, accelerometers, gyroscopes, motion-sensing devices, magnetometers, inertial components for consumer electronics, etc. Thus, the teachings are not intended to be limited to the implementations depicted solely in the Figures, but instead have wide applicability as will be readily apparent to one having ordinary skill in the art.

As used herein, a phrase referring to "at least one of" a list of items refers to any combination of those items, including single members. As an example, "at least one of: a, b, or c" is intended to cover: a, b, c, a-b, a-c, b-c, and a-b-c.

The various illustrative logics, logical blocks, modules, circuits and algorithm processes described in connection with the implementations disclosed herein may be implemented as electronic hardware, computer software, or combinations of both. The interchangeability of hardware and software has been described generally, in terms of functionality, and illustrated in the various illustrative components, blocks, modules, circuits and processes described above. Whether such functionality is implemented in hardware or software depends upon the particular application and design constraints imposed on the overall system.

The hardware and data processing apparatus used to implement the various illustrative logics, logical blocks, modules and circuits described in connection with the aspects disclosed herein may be implemented or performed with a general purpose single- or multi-chip processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor may be a microprocessor, or, any conventional processor, controller, microcontroller, or state machine. A processor also may be implemented as a combination of computing devices, e.g., a combination of a DSP and a microprocessor, a plurality of microprocessors, one or more microprocessors in conjunction with a DSP core, or any other such configuration. In some implementations, particular processes and methods may be performed by circuitry that is specific to a given function.

In one or more aspects, the functions described may be implemented in hardware, digital electronic circuitry, computer software, firmware, including the structures disclosed in this specification and their structural equivalents thereof, or in any combination thereof. Implementations of the subject matter described in this specification also may be implemented as one or more computer programs, i.e., one or more modules of computer program instructions, encoded on a computer storage media for execution by, or to control the operation of, data processing apparatus.

If implemented in software, the functions may be stored on or transmitted over as one or more instructions or code on a computer-readable medium, such as a non-transitory medium. The processes of a method or algorithm disclosed herein may be implemented in a processor-executable software module which may reside on a computer-readable medium. Computer-readable media include both computer storage media and communication media including any medium that may be enabled to transfer a computer program from one place to another. Storage media may be any available media that may be accessed by a computer. By way of example, and not limitation, non-transitory media may include RAM, ROM, EEPROM, CD-ROM or other optical disk storage, magnetic disk storage or other magnetic storage devices, or any other medium that may be used to store desired program code in the form of instructions or data structures and that may be accessed by a computer. Also, any connection may be properly termed a computer-readable medium. Disk and disc, as used herein, includes compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and instructions on a machine readable medium and computer-readable medium, which may be incorporated into a computer program product.

Various modifications to the implementations described in this disclosure may be readily apparent to those having ordinary skill in the art, and the generic principles defined herein may be applied to other implementations without departing from the spirit or scope of this disclosure. Thus, the disclosure is not intended to be limited to the implementations shown herein, but is to be accorded the widest scope consistent with the claims, the principles and the features disclosed herein. The word "exemplary" is used exclusively herein, if at all, to mean "serving as an example, instance, or illustration." Any implementation described herein as "exemplary" is not necessarily to be construed as preferred or advantageous over other implementations. The disclosure should not be limited except in accordance with the claims.

Certain features that are described in this specification in the context of separate implementations also may be implemented in combination in a single implementation. Conversely, various features that are described in the context of a single implementation also may be implemented in multiple implementations separately or in any suitable subcombination. Moreover, although features may be described above as acting in certain combinations and even initially claimed as such, one or more features from a claimed combination may in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the implementations described above should not be understood as requiring such separation in all implementations, and it should be understood that the described program components and systems may generally be integrated together in a single software product or packaged into multiple software products. Additionally, other implementations are within the scope of the following claims. In some cases, the actions recited in the claims may be performed in a different order and still achieve desirable results.

It will be understood that unless features in any of the particular described implementations are expressly identified as incompatible with one another or the surrounding context implies that they are mutually exclusive and not readily combinable in a complementary and/or supportive sense, the totality of this disclosure contemplates and envisions that specific features of those complementary implementations may be selectively combined to provide one or more comprehensive, but slightly different, technical solutions. It will therefore be further appreciated that the above description has been given by way of example only and that modifications in detail may be made within the scope of this disclosure.

### EMBODIMENT CLAUSES

1. A breathprint sensor system for verifying an identity of a person, the system comprising: one or more first sensors having first response characteristics to compounds in gases, the one or more first sensors being configured to output sensor data representing the first response characteristics; and one or more processors in communication with the one or more first sensors, the one or more processors being configured to: receive a set of test data provided by the one or more first sensors based on an exposure of the one or more first sensors to gases produced by the person during a test phase; and determine whether the set of test data verifies the identity of the person.
2. The breathprint sensor system of clause 1, further comprising: one or more second sensors having second response characteristics to compounds in gases, the one or more second sensors being configured to output supplemental sensor data representing the second response characteristics; the one or more processors being in further communication with the one or more second sensors and being further configured to: receive a set of supplemental sensor data provided by the one or more second sensors based on an exposure of the one or more second sensors to the gases produced by the person during the test phase; identify a biological or environmental condition associated with the set of supplemental sensor data; and provide information indicating the biological or environmental condition.
3. The breathprint sensor system of clause 1 or clause 2, wherein determining whether the set of test data verifies the identity of the person comprises: pre-processing the test data; and using a pattern classifier to classify the pre-processed test data into one of a plurality of classes comprising: (i) identity verified when a pattern of the test data is recognized as belonging to the person, and (ii) identity not verified when a pattern of the test data is recognized as not belonging to the person.
4. The breathprint sensor system of clause 3, wherein the pattern classifier comprises a neural network pattern classifier.
5. The breathprint sensor system of clause 3 or clause 4, wherein the one or more processors are further configured to train the pattern classifier from one or more sets of training data.
6. The breathprint sensor system of clause 5, wherein training the pattern classifier from one or more sets of training data comprises: receiving one or more sets of positive training data provided by the one or more first sensors based on one or more exposures of the one or more first sensors to gases produced by the person during a training phase; providing the one or more sets of positive training data to the pattern classifier; and informing the pattern classifier that the one or more sets of positive training data belong to the person.
7. The breathprint sensor system of clause 6, wherein training the pattern classifier from one or more sets of training data further comprises: receiving one or more sets of negative training data provided by the one or more first sensors based on one or more exposures of the one or more first sensors to gases not produced by the person during a training phase; providing the one or more sets of negative training data to the pattern classifier; and informing the pattern classifier that the one or more sets of negative training data do not belong to the person.
8. The breathprint sensor system of any one of the preceding clauses, wherein the one or more first sensors are made from sensor materials selected from the group consisting of: conducting polymer, conducting polymer composites, intrinsically conducting polymers, and any combinations thereof.
9. The breathprint sensor system of any one of the preceding clauses, the one or more first sensors being further configured to obtain the sensor data when a surface temperature of the one or more first sensors is unmodulated.
10. The breathprint sensor system of any one of the preceding clauses, further comprising a memory configured to store the sensor data and/or information derived from the sensor data.
11. The breathprint sensor system of any one of the preceding clauses, wherein each sensor comprises a polymer layer having a variable conductance based on exposure to volatile organic compounds (VOCs) in gases.
12. The breathprint sensor system of any one of the preceding clauses, wherein the one or more processors are further configured to: derive a test feature vector from the test data through feature extraction.
13. The breathprint sensor system of clause 12, wherein the one or more processors are further configured to: compare the test feature vector to a training feature vector derived from training data; and based on the comparison, determine whether the test data verifies the identity of the person.
14. A smart inhaler system for delivering drugs to a person by inhalation, the system comprising: a breathprint sensor system configured to verify an identity of the person using gases produced by the person; an inhaler apparatus adapted to deliver the drugs to the person through inhalation when the inhaler apparatus is received by the person; and a control system in communication with the breathprint sensor system and with the inhaler apparatus, the control system configured to: receive information from the breathprint sensor system, the information indicating whether the person's identity is verified, and control an operation of the inhaler apparatus according to the received information.
15. The smart inhaler system of clause 14, wherein controlling the operation of the inhaler apparatus according to the received information comprises controlling a delivery of a drug according to the received information.
16. The smart inhaler system of clause 14 or clause 15, further comprising an interface system for inputting data to and/or outputting data from the breathprint sensor system, the inhaler apparatus, and/or the control system.
17. The smart inhaler system of clause 16, wherein the interface system comprises a wireless network interface for exchanging data with an external device via a wireless network.
18. The smart inhaler system of clause 16 or clause 17, wherein the interface system comprises an input/output device configured to receive user inputs and provide information to users.
19. The smart inhaler system of clause 18, wherein the input/output device comprises one selected from the group consisting of: a display device, a light emitting diode, a speaker, a touch sensitive input device, a button, a haptic device, and any combinations thereof.
20. The smart inhaler system of any one of clauses 14-19, wherein the control system is further configured to send a notification indicating that an unverified person attempted to use the inhaler apparatus when the information received from the breathprint sensor system indicates that the person's identity is not verified.
21. The smart inhaler system of any one of clauses 14-20, wherein the control system is further configured to deactivate the inhaler apparatus when the information received from the breathprint sensor system indicates that the person's identity is not verified.
22. The smart inhaler system of any one of clauses 14-21, wherein the control system is further configured to prompt the person to provide a breath sample to the breathprint sensor system when the information received from the breathprint sensor system indicates that the person's identity is not verified.
23. The smart inhaler system of any one of clauses 14-22, wherein the control system is further configured to prompt the person to provide alternative information that is not derived from a breath sample to verify the person's identity when the information received from the breathprint sensor system indicates that the person's identity is not verified.
24. The smart inhaler system of any one of clauses 14-23, wherein the control system comprises one or more processors communicatively coupled with the breathprint sensor system and the inhaler apparatus.
25. The smart inhaler system of clause 24, wherein at least one of the processors is configured to: analyze information derived from a breath sample produced by the person and determine the person has a breathing problem, associate the breathing problem with one or more environmental conditions, associate the one or more environmental conditions with location data, and create a map of the one or more environmental conditions associated with the breathing problem.
26. The smart inhaler system of any one of clauses 14-25, wherein the breathprint sensor system comprises: one or more first sensors having first response characteristics to compounds in gases, the one or more first sensors configured to output sensor data representing the first response characteristics; and one or more second sensors having second response characteristics to compounds in gases, the one or more second sensors configured to output supplemental sensor data representing the second response characteristics; wherein the first response characteristics are tuned for verifying the identity of the person, and the second response characteristics are tuned for one or more biological markers.
27. The smart inhaler system of clause 26, wherein the one or more biological markers relate to pharmacokinetics of a drug, and wherein at least one of the processors is configured to: determine an efficacy of a dose of the drug delivered by the inhaler apparatus using the supplemental sensor data representing the second response characteristics tuned for the one or more biological markers, and determine a delivery plan of the drug based on the efficacy.
28. A method for verifying an identity of a person using a breathprint sensor system comprising one or more first sensors having first response characteristics to compounds in gases, the method comprising: receiving a set of test data provided by the one or more first sensors based on an exposure of the one or more first sensors to gases produced by the person; and determining whether the set of test data verifies the identity of the person.
29. The method of clause 28, wherein the breathprint sensor system further comprises one or more second sensors having second response characteristics to compounds in gases, the method further comprising: receiving a set of supplemental sensor data provided by the one or more second sensors based on an exposure of the one or more second sensors to the gases produced by the person; identifying a biological or environmental condition associated with the set of supplemental sensor data; and providing information indicating the biological or environmental condition.
30. The method of clause 28 or clause 29, wherein determining whether the set of test data verifies the identity of the person comprises: pre-processing the test data; and using a pattern classifier to classify the pre-processed test data into one of a plurality of classes comprising: (i) identity verified when a pattern of the test data is recognized as belonging to the person, and (ii) identity not verified when a pattern of the test data is recognized as not belonging to the person.
31. The method of clause 30, further comprising, before classifying the pre-processed test data, training the pattern classifier from one or more sets of training data.
32. The method of clause 31, wherein training the pattern classifier comprises: receiving one or more sets of positive training data provided by the one or more first sensors based on one or more exposures of the one or more first sensors to gases produced by the person during a training phase; providing the one or more sets of positive training data to the pattern classifier; and informing the pattern classifier that the one or more sets of positive training data belong to the person.
33. The method of clause 32, wherein training the pattern classifier further comprises: receiving one or more sets of negative training data provided by the one or more first sensors based on one or more exposures of the one or more first sensors to gases not produced by the person during a training phase; providing the one or more sets of negative training data to the pattern classifier; and informing the pattern classifier that the one or more sets of negative training data do not belong to the person.
34. A method for controlling a smart inhaler system comprising a breathprint sensor system, an inhaler apparatus, and one or more processors in communication with the breathprint sensor system and with the inhaler apparatus, the method comprising: receiving information from the breathprint sensor system indicating whether a person's identity is verified using gases produced by the person; and controlling operation of the inhaler apparatus according to the received information.
35. The method of clause 34, wherein controlling the operation of the inhaler apparatus according to the received information comprises controlling a delivery of a drug according to the received information.
36. The method of clause 34 or clause 35, wherein controlling the operation of the inhaler apparatus according to the received information comprises performing, when the information received from the breathprint sensor system indicates that the person's identity is not verified, an operation selected from the group consisting of: sending a notice to another person notifying that an unverified person attempted to use the inhaler; deactivating the inhaler apparatus; prompting the person to provide a breath sample to the breathprint sensor system; prompting the person to provide an alternative information that is not derived from any breath samples to verify the person's identity; and any combinations thereof.
37. A non-transitory computer-readable medium storing computer-readable program code to be executed by one or more processors, the program code comprising instructions to cause a breathprint sensor system comprising one or more first sensors having first response characteristics to compounds in gases to: receive a set of test data provided by the one or more first sensors based on an exposure of the one or more first sensors to gases produced by a person; and determine whether the set of test data verifies an identity of the person.
38. The non-transitory computer-readable medium of clause 37, wherein determining whether the set of test data verifies the identity of the person comprises: pre-processing the test data; and using a pattern classifier to classify the pre-processed test data into one of a plurality of classes comprising: (i) identity verified when a pattern of the test data is recognized as belonging to the person, and (ii) identity not verified when a pattern of the test data is recognized as not belonging to the person.
39. The non-transitory computer-readable medium of clause 37 or clause 38, the program code further comprising instructions to cause the breathprint sensor system to: receive a set of supplemental sensor data provided by one or more second sensors based on an exposure of the one or more second sensors to the gases produced by the person; identify a biological or environmental condition associated with the set of supplemental sensor data; and provide information indicating the biological or environmental condition; wherein the breathprint sensor system further comprises the one or more second sensors having second response characteristics to compounds in gases.
40. A non-transitory computer-readable medium storing computer-readable program code to be executed by one or more processors, the program code comprising instructions configured to cause a smart inhaler system comprising a breathprint sensor system and an inhaler apparatus to: receive information from the breathprint sensor system indicating whether a person's identity is verified using gases produced by the person; and control operation of the inhaler apparatus according to the received information.

## Claims

1. A smart inhaler system for compliance-based delivery of one or more drugs to a person by inhalation, the system comprising:
a breathprint sensor system configured to process gases produced by the person;
an inhaler apparatus configured to deliver the one or more drugs to the person by inhalation; and
a control system configured to:
receive drug dosage data and time data, the drug dosage data identifying a past dosage of the one or more drugs, the time data identifying a time at which the past dosage was delivered to the person, and
control an operation of the inhaler apparatus according to at least the drug dosage data and the time data.

2. The smart inhaler system of claim 1, wherein controlling the operation of the inhaler apparatus comprises:
adjusting a current dosage of the one or more drugs.

3. The smart inhaler system of claim 1, further comprising:
a memory configured to store the drug dosage data in association with the time data and to store compliance data identifying a plurality of past dosages and a plurality of times at which the past dosages were delivered in association with patient identity data identifying the person, the compliance data comprising the drug dosage data and the time data.

4. The smart inhaler system of claim 1, wherein the drug dosage data and the time data are received from a database configured to record compliance data identifying a plurality of past dosages and a plurality of times at which the past dosages were delivered in association with patient identity data identifying the person, the compliance data comprising the drug dosage data and the time data.

5. The smart inhaler system of claim 1, the control system further configured to:
identify a current dosage of the one or more drugs and a current time for delivery of the current dosage to the person.

6. The smart inhaler system of claim 1, the control system further configured to:
compare, with a drug delivery schedule, one or both of:
the drug dosage data and the time data, or
a current dosage of the one or more drugs and a current time for delivery of the current dosage to the person, and
determine, based on the comparison, compliance of the person with the drug delivery schedule, the operation of the inhaler apparatus being further controlled according to the compliance.

7. The smart inhaler system of claim 1, wherein the operation of the inhaler apparatus is further controlled according to one or both of: a biological condition or an environmental condition.

8. A smart inhaler system for compliance-based delivery of one or more drugs to a person by inhalation, the system comprising:
a breathprint sensor system configured to process gases produced by the person, the breathprint sensor system comprising a plurality of sensors having response characteristics to compounds in the gases, the response characteristics being at different levels responsive to exposure of the sensors to a breath sample following an instructed administering of the one or more drugs to the person, the sensors configured to output sensor data representing the different levels of the response;
an inhaler apparatus configured to deliver the one or more drugs to the person by inhalation; and
a control system configured to:
receive the sensor data; and
determine, based on the sensor data, one or both of:
a dosage of the one or more drugs to the person, or
a time for administering the dosage to the person.

9. The smart inhaler system of claim 8, the control system further configured to control one or both of:
operation of the inhaler apparatus according to at least the dosage and the time, the operation comprising administering of the dosage to the person, or
output of an alert according to the sensor data.

10. The smart inhaler system of claim 8, the control system further configured to:
compare, with a drug delivery schedule, the dosage and the time,
determine, based on the comparison, compliance of the person with the drug delivery schedule, and
control operation of the inhaler apparatus, at least in part, according to the compliance.

11. The smart inhaler system of claim 8, the sensors configured to detect a change of one or more markers related to pharmacokinetics of the one or more drugs.

12. The smart inhaler system of claim 8, wherein each sensor comprises a polymer layer having a variable conductance based on exposure to volatile organic compounds, VOCs, in gases.

13. The smart inhaler system of claim 8, further comprising:
an interface system providing communication among at least the breathprint sensor system, the inhaler apparatus, and the control system, the interface system comprising a wireless network interface for exchanging data with an external device via a wireless network.

14. A non-transitory computer-readable medium storing program code to be executed by one or more processors associated with a breathprint sensor system configured to process gases produced by a person and associated with an inhaler apparatus configured to deliver one or more drugs to the person by inhalation, the program code comprising instructions configured to cause:
processing drug dosage data and time data, the drug dosage data identifying a past dosage of the one or more drugs, the time data identifying a time at which the past dosage was delivered to the person; and
controlling an operation of the inhaler apparatus according to at least the drug dosage data and the time data.

15. A method of determining a dosage and a time for administering the dosage of one or more drugs to a person by inhalation in association with a breathprint sensor system configured to process gases produced by the person, the breathprint sensor system comprising a plurality of sensors having response characteristics to compounds in the gases, the response characteristics being at different levels responsive to exposure of the sensors to a breath sample following an instructed first administering of the one or more drugs to the person, the sensors configured to output sensor data representing the different levels of the response, and in association with an inhaler apparatus configured to deliver the one or more drugs to the person by inhalation, the method comprising:
receiving the sensor data; and
determining, based on the sensor data, one or both of:
a dosage of the one or more drugs to the person, or
a time for administering the dosage to the person; and
controlling one or both of:
operation of the inhaler apparatus according to at least the dosage and the time, or
output of an alert according to the sensor data.
